# EUROPEAN PATENT APPLICATION

(11) **EP 4 498 327 A1**
(43) Date of publication of application: **29.01.2025**
(21) Application number: 24191189.0
(22) Date of filing: 26.07.2024
(51) Int. Cl.: G06V 10/22, G06T 7/00, G06V 10/20, G06V 30/146, G16H 50/20

(54) **APPARATUS AND METHOD FOR ASSISTING FINDING REGION MATCHING IN MEDICAL IMAGES**

(30) Priority: 27.07.2023 KR 20230098226
(71) Applicant: Coreline Soft Co., Ltd, Seoul 03991 (KR)
(72) Inventor: KWON, Sunggoo, 11902 Gyeonggi-do (KR); KIM, Hyunwoo, 04413 Seoul (KR); PARK, Chanmi, 03987 Seoul (KR)
(74) Representative: Stöckeler, Ferdinand

(57) **Abstract**

Disclosed herein is a method of assisting finding region matching in medical images, the method comprising: receiving a second medical image generated at a different time and containing anatomical information about a body part of a patient that is the same as that of a first medical image; receiving a user input for a first finding region, unmatched with second finding regions identified in the second medical image, among first finding regions identified in the first medical image; and for the unmatched first finding region, providing a user with one or more matching finding region candidates among the second finding regions for matching with the unmatched first finding region.

## Description

### CROSS-REFERENCE TO RELATED APPLICATION

This application claims priority based on Korean Patent Application No. 10-2023-0098226 filed on July 27, 2023 which is incorporated herein by reference.

### BACKGROUND

### 1. Technical Field

The present invention relates to technology for processing, analyzing, and visualizing medical images, and more particularly to technology for assisting the process of matching finding regions between a plurality of sets of medical images.

### 2. Description of the Related Art

The contents described in this section merely provide information about the related art of the present invention and do not constitute prior art.

Currently, medical images such as computed tomography (CT) images are widely used to make diagnoses by analyzing lesions. For example, CT images of the chest are frequently used for diagnosis because abnormalities in the inside of the body, such as the lungs, the bronchi, and the heart, can be observed.

Some findings that can be diagnosed through chest CT images are not so easy to read that even radiologists can distinguish their features and shapes only after years of training, with the result that human doctors may easily overlook them. In particular, when the level of difficulty in reading is high, as in the case of a lung nodule, a doctor may not see it even if he or she pays a high degree of attention, which may cause a problem.

In order to assist in the reading of images that humans may easily overlook, the need for computer-aided diagnosis (CAD) has emerged. However, conventional CAD technology only assists doctors in making decisions in a considerably limited area.

The reading of a lesion using CAD may include a process of first specifying a region suspected of being a lesion and evaluating a score (e.g., confidence, malignity, and/or the like) for the region. For example, when a plurality of nodules are found in a lung region, it will be necessary to specify a nodule that is expected to be highly malignant among them and determine a future treatment plan.

However, since there are the plurality of nodules, it is difficult to be aware of a nodule having the highest degree of malignancy among them before reading. Furthermore, in many cases, diagnosis starts to be performed from nodules that are not expected to be malignant or that are not actually malignant, resulting in a decrease in reading efficiency. In addition, since it is difficult to know which nodule is an actual nodule before reading and reliability is not high, reading efficiency is low even if diagnosis starts to be performed from a part that is not expected to be an actual nodule.

Korean Patent No. 10-1943011 entitled "Method for Assisting Reading of Medical Images of Subject and Apparatus Using the Same," which is a prior art document, discloses a method and an apparatus using the method that can improve reading efficiency by introducing a score evaluation method into a conventional lesion detection system and allowing lesions with the highest score (e.g., reliability, malignancy, and/or the like) to be read first out of detected lesions.

In Korean Patent No. 10-1943011, there is disclosed a configuration in which, when a plurality of lesions are detected for a single type of disease, a list arranged from lesions having the highest score in terms of reliability, malignancy, and/or the like is displayed within a single display setting and an image associated with a corresponding lesion is displayed when a user selects the lesion from the list.

Meanwhile, Korean Patent No. 10-1923962 entitled "Method for Supporting Viewing of Medical Images and Apparatus Using the Same," which is another prior art document, discloses an invention for supporting the easy viewing of chest CT images in a time series, which supports the facilitation of comparison and analysis by showing a time-series set of medical images at the same visual location by using follow-up examination.

Korean Patent No. 10-2283673 entitled "Medical Image Reading Support Apparatus and Method for Adjusting Threshold of Diagnosis Assistance Information Based on Lesion Follow-up Examination," which is still another prior art document, describes an invention for adjusting information about finding regions exposed during lesion follow-up examination.

### DISCLOSURE OF INVENTION

### 1. Technical Problem

In the prior art, in order to support lesion follow-up examination on a time-series set of medical images, it is assumed that automatic matching is performed between finding regions of a baseline image and finding regions of a follow-up image.

In the prior art, the automatic matching of finding regions may fail, or users who are medical experts may have doubts about the results of automatic matching.

When the automatic matching of finding regions fails or the results of automatic matching are not trustworthy in this manner, there may be cases where a user needs to match finding regions manually or semi-automatically.

The present invention has been conceived to overcome the problems of the prior art, and an object of the present invention is to provide an apparatus and method that efficiently assist the process of matching finding regions in a time-series set of medical images.

An object of the present invention is to propose a user interface that sorts and visualizes matching finding region candidates based on their suitability for matching between finding regions.

An object of the present invention is to propose a criterion for efficiently sorting optimal matching finding region candidates for matching between finding regions.

### 2. Technical Solution

According to an aspect of the present invention, there may be provided a method of assisting finding region matching in medical images, the method comprising: receiving a second medical image generated at a different time and containing anatomical information about a body part of a patient that is the same as that of a first medical image; receiving a user input for a first finding region, unmatched with second finding regions identified in the second medical image, among first finding regions identified in the first medical image; and for the unmatched first finding region, providing a user with one or more matching finding region candidates among the second finding regions for matching with the unmatched first finding region, wherein the providing comprises providing the one or more matching finding region candidates, sorted based on at least one of 1) locations, 2) sizes, or 3) characteristics of the one or more matching finding region candidates, to the user.

In the method of the present invention, the location of each of the one or more matching finding region candidates may comprise at least one of 1) a representative slice image in which the matching finding region candidate is located in the second medical image, or 2) a representative location of the matching finding region candidate in the representative slice image.

In the method of the present invention, the characteristic of each of the one or more matching finding region candidates may comprise at least one of 1) a correlation between a shape of the unmatched first finding region and a shape of the matching finding region candidate, or 2) a correlation between clinical diagnosis information of the unmatched first finding region and clinical diagnosis information of the matching finding region candidate.

In the method of the present invention, the clinical diagnosis information of the unmatched first finding region and the clinical diagnosis information of the matching finding region candidate may comprise a Reporting & Data System (RADS) category.

In the method of the present invention, the clinical diagnosis information of the unmatched first finding region and the clinical diagnosis information of the matching finding region candidate may comprise distribution information about a distribution of signal intensity values within a finding region.

The method of the present invention may further comprise, when the user input for the unmatched first finding region is received, providing a user interface through which the user can select a matching command for the unmatched first finding region.

In the method of the present invention, the providing may comprise, when a user input for the matching command is received via the user interface through which the matching command for the unmatched first finding region can be selected, providing the one or more matching finding region candidates to the user in response to the user input for the matching command.

The method of the present invention may further comprise, when an automatic matching process for the first finding regions and the second finding regions fails, visualizing the first finding region, unmatched with the second finding regions, among the first finding regions and providing it to the user.

The method of the present invention may further comprise, providing the user with a first finding region, matched with any one of the second finding regions, among the first finding regions; and providing a user interface through which the user can select an unmatching command for the matched first finding region.

In the method of the present invention, the receiving the user input may comprise providing the user with the unmatched first finding region generated by the unmatching command.

According to an aspect of the present invention, there may be provided an apparatus for assisting finding region matching in medical images, the apparatus comprising: memory configured to store one or more instructions; and a processor configured to execute the one or more instructions, wherein the processor, by executing the one or more instructions, may be configured to: receive a second medical image generated at a different time and containing anatomical information about a body part of a patient that is the same as that of a first medical image; receive a user input for a first finding region, unmatched with second finding regions identified in the second medical image, among first finding regions identified in the first medical image; for the unmatched first finding region, provide a user with one or more matching finding region candidates among the second finding regions for matching with the unmatched first finding region; and provide the one or more matching finding region candidates, sorted based on at least one of 1) locations, 2) sizes, or 3) characteristics of the one or more matching finding region candidates, to the user.

In the apparatus of the present invention, the location of each of the one or more matching finding region candidates may comprise at least one of 1) a representative slice image in which the matching finding region candidate is located in the second medical image, or 2) a representative location of the matching finding region candidate in the representative slice image.

In the apparatus of the present invention, the characteristic of each of the one or more matching finding region candidates may comprise at least one of 1) a correlation between a shape of the unmatched first finding region and a shape of the matching finding region candidate, or 2) a correlation between clinical diagnosis information of the unmatched first finding region and clinical diagnosis information of the matching finding region candidate.

In the apparatus of the present invention, the clinical diagnosis information of the unmatched first finding region and the clinical diagnosis information of the matching finding region candidate may comprise a Reporting & Data System (RADS) category.

In the apparatus of the present invention, the clinical diagnosis information of the unmatched first finding region and the clinical diagnosis information of the matching finding region candidate may comprise distribution information about a distribution of signal intensity values within a finding region.

In the apparatus of the present invention, the processor, by executing the one or more instructions: when the user input for the unmatched first finding region is received, may provide a user interface through which the user can select a matching command for the unmatched first finding region.

In the apparatus of the present invention, the processor, in providing the one or more matching finding region candidates to the user, when a user input for the matching command is received via the user interface through which the matching command for the unmatched first finding region can be selected, may provide the one or more matching finding region candidates to the user in response to the user input for the matching command.

In the apparatus of the present invention, the processor, by executing the one or more instructions: when an automatic matching process for the first finding regions and the second finding regions fails, may visualize the first finding region, unmatched with the second finding regions, among the first finding regions and provides it to the user.

In the apparatus of the present invention, the processor, by executing the one or more instructions: may provide the user with a first finding region, matched with any one of the second finding regions, among the first finding regions; and may provide a user interface through which the user can select an unmatching command for the matched first finding region.

In the apparatus of the present invention, the processor, by executing the one or more instructions, may provide the user with the unmatched first finding region generated by the unmatching command.

### 3. Advantageous Effects

According to an embodiment of the present invention, it may be possible to efficiently assist the process of matching finding regions in a time-series set of medical images.

According to an embodiment of the present invention, it may be possible to provide the user interface that sorts and visualizes matching finding region candidates based on their suitability for matching between finding regions.

According to an embodiment of the present invention, it may be possible to provide the criterion for efficiently sorting optimal matching finding region candidates for matching between finding regions.

### DESCRIPTION OF THE DRAWINGS

The above and other objects, features, and advantages of the present invention will be more clearly understood from the following detailed description taken in conjunction with the accompanying drawings, in which:
FIG. 1 is a conceptual diagram illustrating a user interface in which a method of performing finding region matching in medical images according to an embodiment of the present invention is provided;
FIG. 2 is an operational flowchart showing a method of performing finding region matching in medical images according to an embodiment of the present invention;
FIG. 3 is a conceptual diagram showing an embodiment of a user interface corresponding to steps S220 and S230 in the process of FIG. 2;
FIG. 4 is a conceptual diagram showing an embodiment of a user interface corresponding to step S250 in the process of FIG. 2;
FIG. 5 is a conceptual diagram showing an embodiment of the clinical diagnosis of a matching finding region candidate as part of the process of FIGS. 2 and 3;
FIG. 6 is a conceptual diagram showing another embodiment of the clinical diagnosis of a matching finding region candidate as part of the process of FIGS. 2 and 3;
FIG. 7 is an operational flowchart showing a method of performing finding region unmatching in medical images according to an embodiment of the present invention;
FIG. 8 is a conceptual diagram showing an embodiment of a user interface corresponding to the process of FIG. 7;
FIG. 9 is a conceptual diagram showing an embodiment of the unmatching menu 150, which is a user interface corresponding to step S420 in the process of FIG. 7; and
FIG. 10 is a conceptual diagram showing an example of a generalized medical image processing, analysis, visualization, diagnosis assistance, and finding region matching assistance apparatus or computing system capable of performing at least part of the processes of FIGS. 1 to 9.

### DETAILED DESCRIPTION

Other objects and features of the present invention in addition to the above-described objects will be apparent from the following description of embodiments to be given with reference to the accompanying drawings.

The embodiments of the present invention will be described in detail below with reference to the accompanying drawings. In the following description, when it is determined that a detailed description of a known component or function may unnecessarily make the gist of the present invention obscure, it will be omitted.

Relational terms such as first, second, and the like may be used for describing various elements, but the elements should not be limited by the terms. These terms are only used to distinguish one element from another. For example, a first component may be named a second component without departing from the scope of the present disclosure, and the second component may also be similarly named the first component. The term "and/or" means any one or a combination of a plurality of related and described items.

When it is mentioned that a certain component is "coupled with" or "connected with" another component, it should be understood that the certain component is directly "coupled with" or "connected with" to the other component or a further component may be disposed therebetween. In contrast, when it is mentioned that a certain component is "directly coupled with" or "directly connected with" another component, it will be understood that a further component is not disposed therebetween.

The terms used in the present disclosure are only used to describe specific exemplary embodiments, and are not intended to limit the present disclosure. The singular expression includes the plural expression unless the context clearly dictates otherwise. In the present disclosure, terms such as 'comprise' or `have' are intended to designate that a feature, number, step, operation, component, part, or combination thereof described in the specification exists, but it should be understood that the terms do not preclude existence or addition of one or more features, numbers, steps, operations, components, parts, or combinations thereof.

Unless otherwise defined, all terms (including technical and scientific terms) used herein have the same meaning as commonly understood by one of ordinary skill in the art to which this disclosure belongs. Terms that are generally used and have been in dictionaries should be construed as having meanings matched with contextual meanings in the art. In this description, unless defined clearly, terms are not necessarily construed as having formal meanings.

Meanwhile, even when the technology was known before the filing date of the present application, it may be included as part of the configuration of the present invention of the present application when necessary, and this will be described herein to the extent that it does not obscure the purpose of the present invention. However, in the following description of the configuration of the present invention of the present application, detailed descriptions of items that can be clearly understood by those skilled in the art as the technologies known before the filing date of the present application may obscure the purpose of the present invention, so that excessively detailed descriptions of known technologies will be omitted.

For example, technologies known before the application of the present invention may be used as technology for detecting, segmenting, and classifying specific organs and sub-regions of the human body by processing medical images, technology for generating quantified information by measuring segmented organs or finding regions, and the like, and at least some of these known technologies may be applied as elemental technologies required for practicing the present invention. For example, descriptions of elemental technologies required for practicing parts of the configuration of the present invention may be replaced by providing notification that the technologies are known to those skilled in the art via Korean Patent No. 10-2283673 entitled "Medical Image Reading Assistant Apparatus and Method for Adjusting Threshold of Diagnostic Assistant Information Based on Lesion Follow-up Examination," Korean Patent No. 10-1943011 entitled "Method for Assisting Reading of Medical Images of Subject and Apparatus Using the Same," Korean Patent No. 10-1887194 entitled "Method for Assisting Reading of Medical Images of Subject and Apparatus Using the Same," and Korean Patent No. 10-1818074 entitled "Artificial Intelligence-based Medical Automatic Diagnosis Assistant Method and System Thereof."

In the prior art literature, lesion candidates are detected using an artificial neural network, and findings are generated by classifying the lesion candidates. Each of the findings includes diagnosis assistance information. The diagnosis assistance information may include quantitative measurements such as the probability that each finding is actually a lesion, the confidence and malignity of the finding, and the sizes and volumes of lesion candidates to which the finding corresponds.

In medical image diagnosis support using an artificial neural network, each finding needs to include probability and quantified confidence as diagnosis assistance information. Since all findings may not be provided to a user, findings are generally filtered by applying a specific threshold, and only findings that are filtered out are provided to the user. In a workflow in which a clinical finding is generated in such a manner that a user who is a radiologist reads a medical image and the results of diagnosis are generated in such a manner that a clinician analyzes the finding, an artificial neural network or automated program may at least partially assist the radiologist in a reading process and a finding generation process and/or the clinician in a diagnosis process.

However, the purpose of the present invention is not to claim rights to these known technologies, and the content of the known technologies may be included as part of the present invention within the range that does not depart from the spirit of the present invention.

Hereinafter, embodiments of the present invention will be described in more detail with reference to the accompanying drawings. In order to facilitate overall understanding when describing the present invention, the same reference numerals will be used for the same components in the drawings, and redundant descriptions of the same components will be omitted.

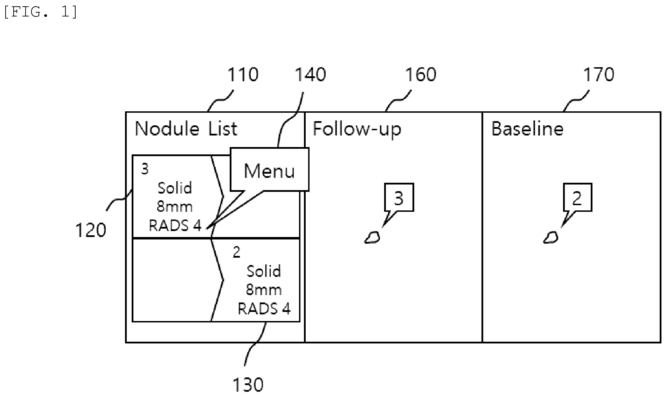

FIG. 1 is a conceptual diagram illustrating a user interface in which a method of performing finding region matching in medical images according to an embodiment of the present invention is provided.

In the prior art, in order to support lesion follow-up examination on a time-series set of medical images, it is assumed that automatic matching is performed between finding regions of a baseline image and finding regions of a follow-up image.

In the prior art, the automatic matching of finding regions may fail, or users who are medical experts may be dissatisfied with automatic matching results.

In this case, even when the results of automatic matching obtained by the automatic matching technology of the prior art or the neural network of the prior art are visualized along with scores such as confidence and malignity by the neural network, it is difficult to know the basis for calculating confidence and/or malignity from the outside because the inside of the neural network is similar to a black box. Accordingly, there is a problem in that it is difficult for users who are medical experts to completely trust the results of automatic matching because they may not be provided with a means for verifying the inference results of the neural network.

When the automatic matching of finding regions fails or the results of automatic matching are not trustworthy, there may be cases where a user needs to match finding regions manually or semi-automatically.

In the present specification, there are mainly described embodiments in which lung nodules are finding regions, but the subject matter of the present invention is not limited to the specific embodiments. The finding region of the present invention may refer to a lesion highly correlated with a specific disease or diagnosis within an anatomical area, such as an organ within a body part, or a region satisfying specific clinically significant conditions.

Referring to FIG. 1, a baseline image 170 generated at a different time and containing anatomical information about the body part of a patient that is the same as that of a follow-up test image 160 is received or acquired and is then displayed through a user interface.

For convenience of description, the follow-up image 160 is described as a first medical image, and the baseline image 170 is described as a second medical image. In another embodiment of the present invention, it will be apparent to those skilled in the art that one of the follow-up image 160 and the baseline image 170 may be the first medical image and the other may be the second medical image..

One or more first finding regions detected in the follow-up image 160 may be shown, and one or more second finding regions detected in the baseline image 170 may be shown.

In a finding region list section 110, the first finding regions and the second finding regions may be shown. In this case, in FIG. 1, in the finding region list section 110, the first finding regions may be shown in a left area 120 and the second finding regions may be shown in a right area 130 according to the arrangement of the follow-up image 160 and the baseline image 170 to intuitively aid in the understanding of a user.

As shown in FIG. 1, when the right side of first finding region "3" in the finding region list section 110 is blank, it means that first finding region "3" is an unmatched finding. When the left side of second finding region "2" is blank, it means that second finding region "2" is an unmatched finding.

As shown in FIG. 1, when a user selects an unmatched finding, a matching menu 140 may be shown. When a specific user input is given for an unmatched finding, it may be considered that the user has selected the unmatched finding. For example, the specific user input may be a left/right mouse click, the operation of positioning a mouse on the icon of the unmatched finding, or the like.

The user input by which a user selects an unmatched finding is recognized as a valid input regardless of whether it is given for an unmatched finding located in any of the finding region list section 110, the follow-up image 160, and/or the baseline image 170. Alternatively, even in the case of a specific user interface action, e.g., the action of dragging an unmatched finding from the follow-up image 160 to the baseline image 170 or the action of dragging toward the follow-up image 160 or the baseline image 170 in the finding region list section 110, a user may be recognized as attempting to match the unmatched finding.

In FIG. 1, only the unmatched findings are shown. However, in another embodiment of the present invention, there may be implemented an embodiment in which pairs of matched findings and unmatched findings are displayed together and the matching menu 140 is invoked in such a manner that a user selects a specific unmatched finding from among the displayed findings.

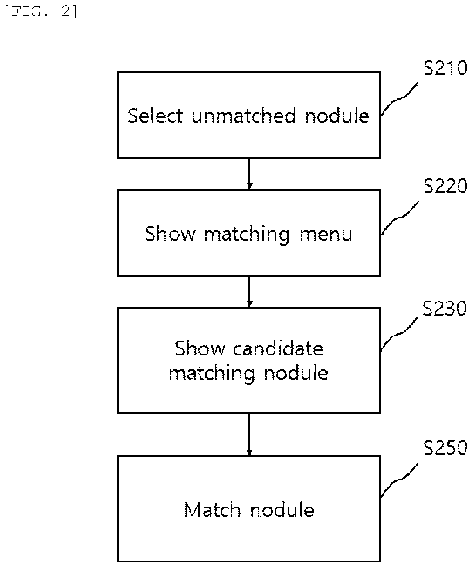

FIG. 2 is an operational flowchart showing a method of performing finding region matching in medical images according to an embodiment of the present invention.

Referring to FIG. 2, a method of assisting finding region matching in medical images according to an embodiment of the present invention includes: step S210 of receiving a user input for a first finding region, unmatched with second finding regions identified in a second medical image, among first finding regions identified in a first medical image; and step S230 of, for the unmatched first finding region, providing a user with one or more matching finding region candidates among the second finding regions for matching with the unmatched first finding region.

Step S230 of providing one or more matching finding region candidates to a user includes the step of providing one or more matching finding region candidates, sorted based on at least one of the 1) locations, 2) sizes, and/or 3) characteristics of the one or more matching finding region candidates, to the user.

For example, the unmatched second finding region closest to the unmatched first finding region selected in the first medical image may start to be sorted and shown. A predetermined number of matching finding region candidates may be shown, and matching finding region candidates for which the matching possibility thereof is equal to or stronger than a predetermined threshold may be shown.

Unmatched second finding regions that may be matched with the unmatched first finding region are shown as matching finding region candidates in the state of being sorted according to a predetermined criterion. Although matching finding region candidates may be shown while having priorities according to a predetermined criterion, the user may select a matching finding region candidate having a lower priority despite the priority.

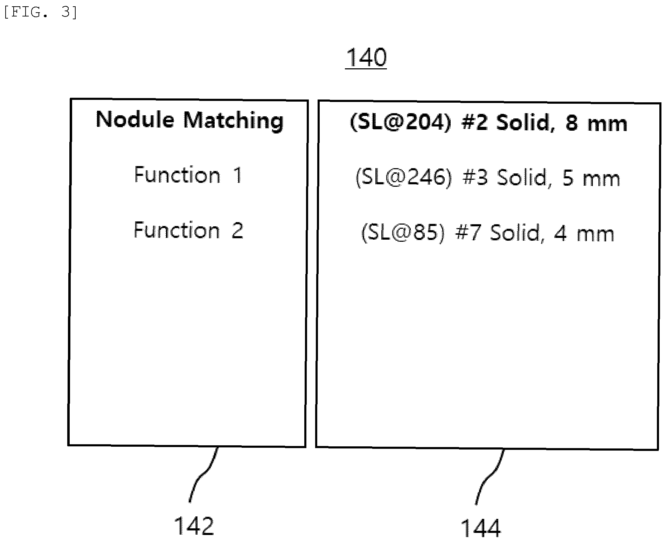

FIG. 3 is a conceptual diagram showing an embodiment of a user interface corresponding to steps S220 and S230 in the process of FIG. 2.

The method of assisting finding region matching in medical images according to the embodiment of the present invention may further include step S220 of, when a user input for an unmatched first finding region is received, providing a user interface through which the user may select a matching command for the unmatched first finding region.

In this case, step S230 of providing one or more matching finding region candidates to the user may include, when a user input for a matching command is received via a user interface through which the matching command for the first unmatched finding region can be selected, providing the one or more matching finding region candidates to the user in response to the user input for a matching command, as shown in the matching finding region candidate list section 144 of FIG. 3.

When the user selects the unmatched first finding region in step S210, an apparatus for assisting finding region matching according to the present invention may provide a number of functions using a menu function section 142. Among the functions provided in this case, a finding matching command may be provided within the menu function section 142.

The location of each of the one or more matching finding region candidates may include at least one of 1) a representative slice image in which the matching finding region candidate is located in the second medical image and/or 2) the representative location of the matching finding region candidate in the representative slice image.

In this case, the representative slice image may be a slice image where the center of the matching finding region candidate is located, and the representative location may be the location of the center of the matching finding region candidate. The center may be the center of gravity, and may be determined by combining one or more of the average, mode, and median of the locations of voxels within the area of the matching finding region candidate.

The matching finding region candidates may be shown along with SL# (the number/location of the slice image), # (index), type, and/or size, as shown in the matching finding region candidate list section 144 of FIG. 3.

When the matching finding region candidates for the unmatched first finding region are provided based on the locations, the similarity of the locations may be determined based on registration between the first medical image and the second medical image. When the results of the registration are used, a relative location in the second medical image that corresponds to the location of the center point of the unmatched first finding region in the first medical image may be identified, and the candidate matching finding regions sorted in order of being closest to the identified relative location in the second medical image may be shown.

When the results of the registration cannot be used, a relative location in the second medical image corresponding to a location in the first medical image may be identified using the results of segmentation of an anatomical region. For example, when the anatomical region is a lung region, the five lobes of the lung may be segmented in detail, and the relative location may be identified based on the results of the detailed segmentation.

The matching finding region candidates may be shown along with locations according to detailed segmentation results for sub-regions in the anatomical region.

The characteristic of each of the one or more matching finding region candidates may include at least one of 1) the correlation between the shape of the unmatched first finding region and the shape of the matching finding region candidate and/or 2) the correlation between the clinical diagnosis information of the unmatched first finding region and the clinical diagnosis information of the matching finding region candidate.

The clinical diagnosis information of the unmatched first finding region and the clinical diagnosis information of each of the one or more matching finding region candidates may include a Lung-Reporting & Data System (RADS) category or a Bi-RADS (Breast Imaging RADS) category.

When a finding region to be diagnosed is a lung nodule, a Lung RADS category may range from 0 to 4, with 4 being classified as 4A, 4B, or 4X depending on the likelihood of malignancy. When a finding region to be diagnosed is a lesion in the breast region, a Bi-RADS category may be included.

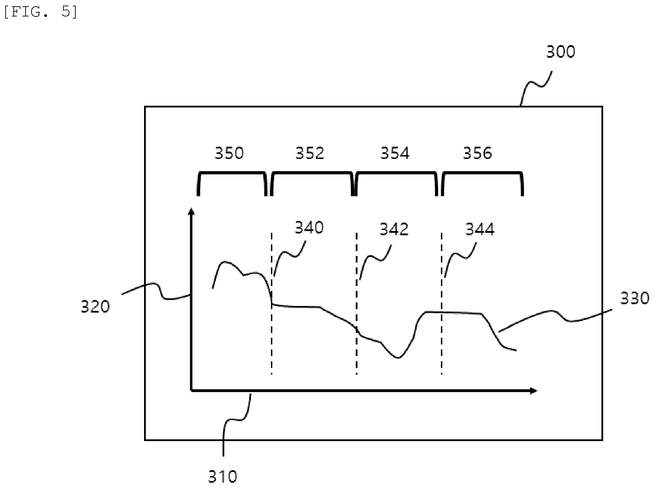

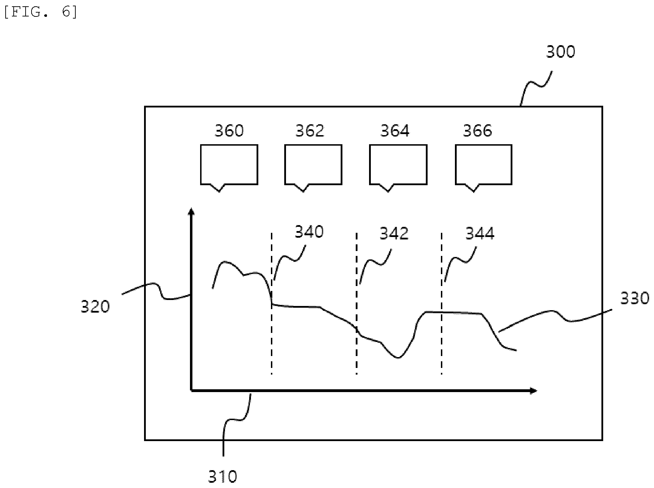

The clinical diagnosis information of the unmatched first finding region and the clinical diagnosis information of each of the one or more matching finding region candidates may include distribution information about the distribution of signal intensity values in a finding region. In this case, the distribution of signal intensity values may be a histogram of the signal intensity values. Details of this embodiment will be described later using FIGS. 5 and 6.

The method of assisting finding region matching in medical images according to the embodiment of the present invention may further include the step of, when the automatic matching process for the first finding regions and the second finding regions fails, visualizing the first finding region, unmatched with the second finding regions, among the first finding regions and providing it to the user.

Examples of the case where automatic matching fails or is not trustworthy may include a case where registration between the first medical image and the second medical image fails or an error is large, a case where incorrect matching occurs when there are a plurality of second finding regions near a relative location in the second medical image corresponding to the location of each of the first finding regions, and the like.

Additionally, automatic matching may fail or may not be trustworthy in cases where it is difficult to match the first medical image and the second medical image or it is difficult to identify a relative location due to segmentation.

According to an embodiment of the present invention, manual or semi-automatic matching between finding areas may be assisted. In this case, effective matching may be assisted by additionally providing indicators indicative of similarity in location, size, and clinical diagnosis so that a user can determine suitability for matching finding region candidates.

When the user selects any one of the matching finding region candidates, the unmatched first finding region and the selected second finding region are matched with each other in step S250.

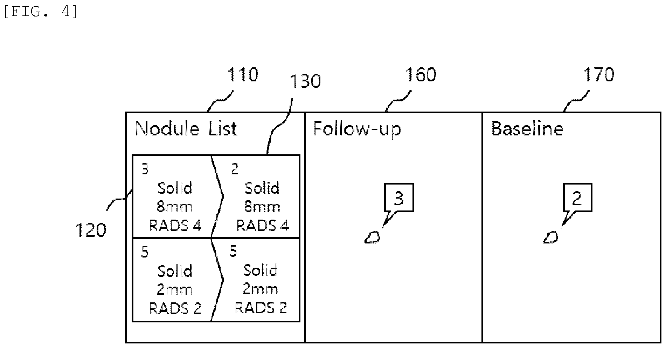

FIG. 4 is a conceptual diagram showing an embodiment of a user interface corresponding to step S250 in the process of FIG. 2.

Referring to FIG. 4, the matching results obtained by performing step S250 are shown.

It is assumed that first finding area "3" and second finding area "2" are matched with each other in step S250. Additionally, a state in which first finding area "5" and second finding area "5" are matched with each other by automatic matching or step S250 may also be shown.

FIG. 5 is a conceptual diagram showing an embodiment of the clinical diagnosis of a matching finding region candidate as part of the process of FIGS. 2 and 3.

FIG. 6 is a conceptual diagram showing another embodiment of the clinical diagnosis of a matching finding region candidate as part of the process of FIGS. 2 and 3.

Referring to FIGS. 5 and 6, the clinical diagnosis information of the unmatched first finding region and the clinical diagnosis information of each of the one or more matching finding region candidates may include distribution information about the distribution of signal intensity values in a finding region. In this case, the distribution of signal intensity values may be a histogram of the signal intensity values.

According to an embodiment of the present invention, the apparatus: may acquire information about a finding region in the first medical image 100; may acquire distribution information 330 about the distribution of signal intensity values within the finding region; may generate first assistant information 340 based on a first threshold for the signal intensity values within the finding region; and may generate second assistant information 300 based on the distribution information 330 and the first assistant information 340.

The distribution information 330 may be visualized along with a horizontal axis 310 corresponding to signal intensity values and a vertical axis 320 corresponding to the numbers and/or distributions of pixels/voxels having signal intensity values. The signal intensity values may be the brightness values of a CT scan image or an X-ray image, and may be expressed in Hounsfield units (HU). The distribution information 330 may be a histogram corresponding to the signal intensity values of pixels/voxels in the finding region.

According to an embodiment of the present invention, the finding region may be a lesion. For example, the finding region may be a lung nodule, a coronary artery calcification (CAC) region, or a lung tumor.

When the finding region is a lung nodule, a user is assisted with a process of identifying the type of nodule by marking -100 HU and 100 HU reference lines as first assistant information 340 along with distribution information 330 for CT brightness values. The range of HU values on a horizontal axis plotted on a histogram is an embodiment specific to a corresponding lesion, and may be optimized within the interval of [-1000 HU, 500 HU] in the case of a lung nodule, for example.

In an embodiment in which the distribution of brightness values is visualized through a histogram, a user may be assisted in identifying the type or state of a finding region by providing one or more reference lines for brightness values. In this case, the results of the quantitative analysis of a plurality of brightness value intervals divided by one or more reference lines may be visualized together with the reference lines.

Alternatively, a medical image analysis module may determine the type or state of a finding region based on one or more reference lines, and the results of the determination may be visualized along with the reference lines. In this case, the results of the quantitative analysis of a plurality of brightness value intervals divided by one or more reference lines may be visualized together with the reference lines and the results of determination of the type or state of a finding region. In this case, the results of the determination may further include predictive/inferential information about a change in the type or state of a finding region.

For example, when the finding region is a lung nodule, a brightness value reference line that makes it easy to identify the type (solid, non-solid, or part-solid) of nodule may be provided.

The first assistant information 340 may be a reference line or an interval mark corresponding to a first threshold visualized together with the distribution information 330. The first threshold may be a threshold for signal intensity values.

The signal intensity values within the finding region may be classified into a first interval 350 corresponding to a first state and a second interval 352 corresponding to a second state based on the first threshold.

The second assistant information 300 may include at least one of first interval distribution information corresponding to the first interval 350 of the signal intensity values within the finding region and second interval distribution information corresponding to the second interval 352 of the signal intensity values within the finding region.

The first interval distribution information may be a segment or part belonging to the first interval 350 of the distribution information 330. The second interval distribution information may be a segment or part belonging to the second interval 352 of the distribution information 330.

The second assistant information 300 may include individual pieces of interval distribution information obtained by dividing the distribution information 330, which is a histogram, into individual intervals 350, 352, 354, and 356, and may also include first assistant information 340, 342, and 344 corresponding to thresholds for division into the individual intervals 350, 352, 354, and 356. The second assistant information 300 may be generated as visualization information in order to be provided to a user through a display or user interface. As the visualization information is visualized to the user, the second assistant information 300 may be provided to the user.

The second assistant information 300 may further include a first visualization element representing the first interval distribution information and a second visualization element representing the second interval distribution information. In other words, individual segments may be visualized using different visualization elements so that a histogram can be visually divided into individual intervals. The visualization elements may include colors, patterns, markers, line thicknesses, solid/dotted lines, and/or shapes.

The first threshold may be a value associated with at least one of the presence/absence, progress, and severity of a disease associated with the finding region. The first threshold may be a value applied to both the finding region and a second finding region 210.

The information about the finding region may include segmentation information about the boundary of the finding region. In other words, the information about the finding region may include boundary information obtained as a result of segmentation.

For example, the finding region may include a nodule, a CAC, a tumor, and the like. For example, the finding may include a low attenuation area (LAA) associated with a disease such as chronic obstructive pulmonary disease (COPD).

An embodiment of the present invention may provide distribution information, first assistant information, and second assistant information to a user as means for verifying information about a finding region, which is an inference result of an artificial neural network or an operation result of a rule-based analysis engine.

In this case, an embodiment of the present invention may generate second assistant information (including distribution information and first assistant information) as visualization information to be provided to a user.

In this case, an embodiment of the present invention may provide the visualization information to the user via a user interface such as a display.

An embodiment of the present invention may provide a user with second assistant information together with visualization information about a finding region in a medical image.

In this case, an embodiment of the present invention may automatically provide a user with second assistant information together with visualization information about a finding region, or may provide a user with second assistant information about a finding region in response to a user input when the user input is recognized for visualization information about the finding region.

In an embodiment of the present invention, the signal intensity values within the finding region may be classified into a first interval corresponding to the first state of the pixels/voxels within the finding region or a second interval corresponding to the second state thereof. In this case, the first state and the second state may be states distinguished from each other in association with at least one of the presence/absence, progress, and severity of a disease associated with the finding region. The first state and the second state may be states that each of the pixels/voxels within the finding region may have, and may be states distinguished from each other in association with at least one of the presence/absence, progress, and/or severity of a disease.

Referring to FIG. 5, the apparatus may generate three pieces of first assistant information 340, 342, and 344 using a plurality of thresholds for the signal intensity values within the finding region.

In this case, the signal intensity values within the finding region may be classified into a first interval 350 corresponding to the first state, the second interval 352 corresponding to the second state, and the third interval 354 corresponding to the third state based on one or more thresholds. The individual intervals may be set to correspond to the states of the pixels/voxels within the finding region. The states may be set differently for the type of finding region in association with the presence/absence, progress, and/or severity of a disease associated with the finding region. Interval information associated with the states may be provided as additional diagnostic information for the finding region.

Referring to FIG. 6, the second assistant information 300 may include at least one of first interval distribution quantification information 360, which is the quantification information of a distribution corresponding to the first interval of the signal intensity values of pixels/voxels within a finding region, and second interval distribution quantification information 362, which is the quantification information of a distribution corresponding to the second interval of the signal intensity values of the pixels/voxels within the finding region.

Pieces of interval distribution quantification information 360, 362, 364, and 366 may be pieces of statistical/quantification information for respective intervals of a histogram graph. The number and distribution of pixels/voxels corresponding to each of the intervals, or the ratio of the number of pixels/voxels corresponding to each interval to the number of pixels/voxels in the overall finding region may be included as each of the pieces of distribution quantification information 360, 362, 364, and 366.

The first interval distribution quantification information 360 may include at least one of a percentage, a maximum value, a minimum value, a mean value, a mode value, and a median value associated with the number/distribution of pixels/voxels having signal intensity values corresponding to the first interval within the finding region.

In the same manner, the second interval distribution quantification information 360 may include at least one of a percentage, a maximum value, a minimum value, a mean value, a mode value, and a median value associated with the number/distribution of pixels/voxels having signal intensity values corresponding to the second interval within the finding region.

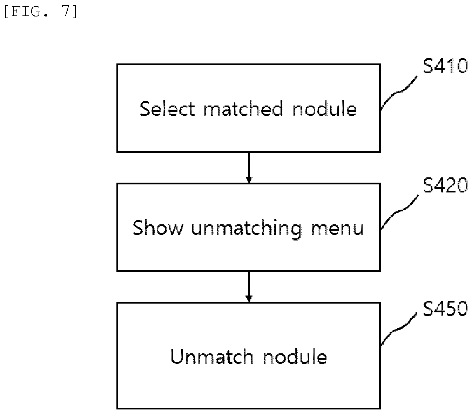

FIG. 7 is an operational flowchart showing a method of performing finding region unmatching in medical images according to an embodiment of the present invention.

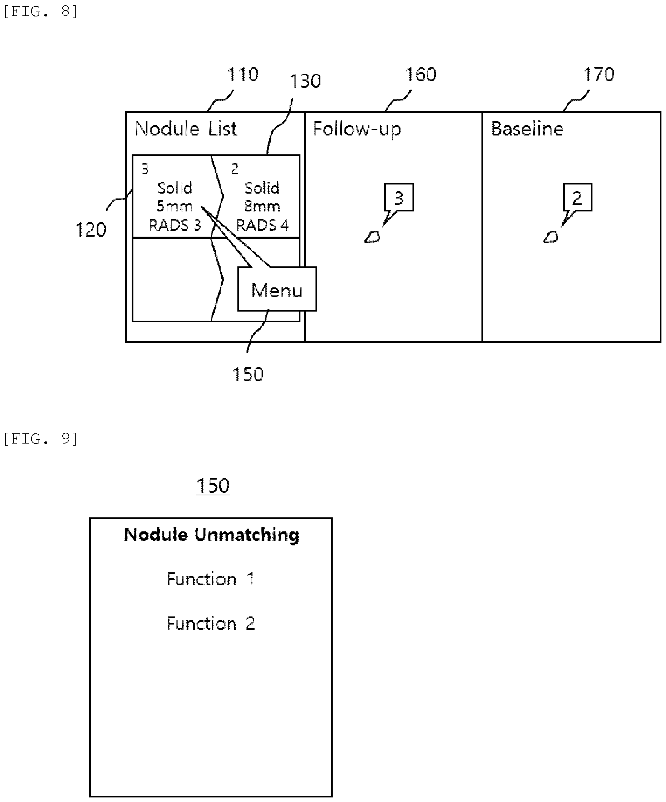

FIG. 8 is a conceptual diagram showing an embodiment of a user interface corresponding to the process of FIG. 7.

Referring to FIGS. 7 and 8 together, the method of assisting finding region matching in medical images according to the embodiment of the present invention may include: step S410 of providing the user with a first finding region, matched with any one of the second finding regions, among the first finding regions; and step S420 of providing a user interface, through which the user can select an unmatching command for the matched first finding region, as an unmatching menu 150. According to an embodiment of the present invention, there may be provided a user interface through which the user unmatches a matched nodule when the user is dissatisfied with the results of automatic matching or re-attempts manual matching.

When the user inputs an unmatching command, the matched first and second finding regions may be unmatched in step S450.

The unmatching command may be given for one matched finding pair or for a plurality of matched finding pairs (the multiple selection of findings is possible).

FIG. 9 is a conceptual diagram showing an embodiment of the unmatching menu 150, which is a user interface corresponding to step S420 in the process of FIG. 7.

The unmatching menu 150 may provide a plurality of function menus for a matched finding region pair. In this case, one of the plurality of function menus may be an unmatching command.

Referring again to FIG. 2, step S210 of receiving a user input for an unmatched first finding region may include the step of providing the user with an unmatched first finding region generated by the unmatching command (not shown). According to an embodiment of the present invention, a nodule unmatched by the user may be re-matched.

According to the embodiment of the present invention disclosed in FIGS. 1 to 9, it may be possible to efficiently assist a finding region matching process in a time-series set of medical images.

According to an embodiment of the present invention, there may be provided a user interface that sorts and visualizes matching finding region candidates based on suitability for matching between finding regions.

According to an embodiment of the present invention, it may be possible to provide a criterion for efficiently sorting optimal matching finding region candidates for matching between finding regions.

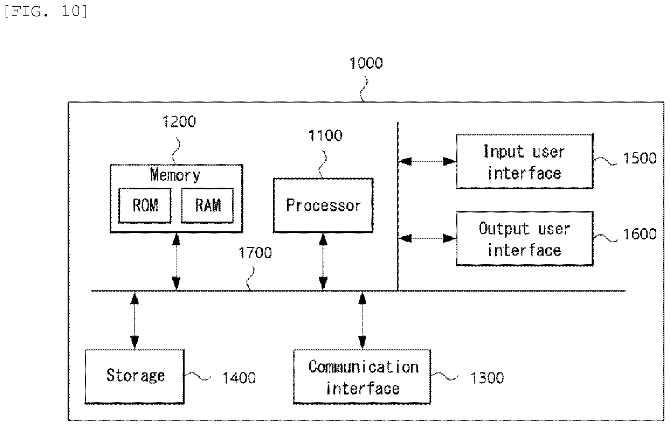

FIG. 10 is a conceptual diagram showing an example of a generalized medical image processing, analysis, visualization, diagnosis assistance, and finding region matching assistance apparatus or computing system capable of performing at least part of the processes of FIGS. 1 to 9.

At least some processes of a method for medical image processing, analysis, visualization, diagnosis assistance, and/or finding region matching assistance according to an embodiment of the present invention may be performed by the computing system 1000 of FIG. 10.

Referring to FIG. 10, the computing system 1000 according to an embodiment of the present invention may include a processor 1100, memory 1200, a communication interface 1300, a storage device 1400, an input interface 1500, an output interface 1600, and a bus 1700.

The computing system 1000 according to an embodiment of the present invention includes at least one processor 1100 and memory 1200 configured to store instructions that instruct the at least one processor 1100 to perform at least one step. At least some steps of the method according to an embodiment of the present invention may be performed in such a manner that the at least one processor 1100 loads instructions from the memory 1200 and executes them.

The processor 1100 may refer to a central processing unit (CPU), a graphics processing unit (GPU), or a dedicated processor on which the methods according to embodiments of the present invention are performed.

Each of the memory 1200 and the storage device 1400 may be formed of at least one of a volatile storage medium and a non-volatile storage medium. For example, the memory 1200 may be formed of at least one of read-only memory (ROM) and random access memory (RAM).

Furthermore, the computing system 1000 may include the communication interface 1300 that performs communication through a wireless network.

Furthermore, the computing system 1000 may further include the storage device 1400, the input interface 1500, and the output interface 1600.

Furthermore, the individual components included in the computing system 1000 may be connected by the bus 1700 and communicate with each other.

Examples of the computing system 1000 of the present invention may include a desktop computer, a laptop computer, a notebook, a smartphone, a tablet personal computer (PC), a mobile phone, a smart watch, smart glasses, an e-book reader, a portable multimedia player (PMP), a portable game console, a car navigation device, a digital camera, a digital multimedia broadcasting (DMB) player, a digital audio recorder, a digital audio player, a digital video recorder, a digital video player, and a personal digital assistant (PDA) that are capable of communication.

An apparatus for assisting finding region matching in medical images according to an embodiment of the present invention may include: memory 1200 configured to store one or more instructions; and a processor 1100 configured to execute the one or more instructions. The processor 1100, by executing the one or more instructions, receives a second medical image generated at a different time and containing anatomical information about the body part of a patient that is the same as that of a first medical image, receives a user input for a first finding region, unmatched with second finding regions identified in the second medical image, among first finding regions identified in the first medical image, and, for the unmatched first finding region, provides a user with one or more matching finding region candidates sorted based on a predetermined condition for matching with the first finding region unmatched among the second finding regions.

The processor 1100, by executing the one or more instructions, may provide the user with one or more matching finding region candidates sorted based on at least one of 1) the locations, 2) sizes, and/or 3) characteristics of the one or more matching finding region candidates.

When a user input for an unmatched first finding region is received, the processor 1100, by executing the one or more instructions, may provide a user interface through which the user can select a matching command for the unmatched first finding region. Furthermore, in the case of providing one or more matching finding region candidates to the user, when a user input for the matching command is received via the user interface through which the user can select the matching command for the unmatched first finding region, the processor 1100, by executing the one or more instructions, may provide one or more matching finding region candidates to the user in response to the user input for the matching command.

When an automatic matching process for first finding regions and second finding region fails, the processor 1100, by executing the one or more instructions, may visualize a first finding region, unmatched with the second finding regions, among the first finding regions and provide it to the user.

The processor 1100, by executing the one or more instructions, may provide the user with a first finding region, matched with any one of second finding regions, among first finding regions, and may also provide a user interface through which the user can select an unmatching command for a matched first finding region.

The processor 1100, by executing the one or more instructions, may provide the user with an unmatched first finding region generated by an unmatching command.

The operations of the method according to the exemplary embodiment of the present disclosure can be implemented as a computer readable program or code in a computer readable recording medium. The computer readable recording medium may include all kinds of recording apparatus for storing data which can be read by a computer system. Furthermore, the computer readable recording medium may store and execute programs or codes which can be distributed in computer systems connected through a network and read through computers in a distributed manner.

The computer readable recording medium may include a hardware apparatus which is specifically configured to store and execute a program command, such as a ROM, RAM or flash memory. The program command may include not only machine language codes created by a compiler, but also high-level language codes which can be executed by a computer using an interpreter.

Although some aspects of the present disclosure have been described in the context of the apparatus, the aspects may indicate the corresponding descriptions according to the method, and the blocks or apparatus may correspond to the steps of the method or the features of the steps. Similarly, the aspects described in the context of the method may be expressed as the features of the corresponding blocks or items or the corresponding apparatus. Some or all of the steps of the method may be executed by (or using) a hardware apparatus such as a microprocessor, a programmable computer or an electronic circuit. In some embodiments, one or more of the most important steps of the method may be executed by such an apparatus.

In some exemplary embodiments, a programmable logic device such as a field-programmable gate array may be used to perform some or all of functions of the methods described herein. In some exemplary embodiments, the field-programmable gate array may be operated with a microprocessor to perform one of the methods described herein. In general, the methods are preferably performed by a certain hardware device.

The description of the disclosure is merely exemplary in nature and, thus, variations that do not depart from the substance of the disclosure are intended to be within the scope of the disclosure. Such variations are not to be regarded as a departure from the spirit and scope of the disclosure. Thus, it will be understood by those of ordinary skill in the art that various changes in form and details may be made without departing from the spirit and scope as defined by the following claims.

## Claims

1. A method of assisting finding region matching in medical images, the method comprising:
receiving a second medical image generated at a different time and containing anatomical information about a body part of a patient that is the same as that of a first medical image;
receiving a user input for a first finding region, unmatched with second finding regions identified in the second medical image, among first finding regions identified in the first medical image; and
for the unmatched first finding region, providing a user with one or more matching finding region candidates among the second finding regions for matching with the unmatched first finding region,
wherein the providing comprises providing the one or more matching finding region candidates, sorted based on at least one of locations, sizes, or characteristics of the one or more matching finding region candidates, to the user.

2. The method of claim 1, wherein the location of each of the one or more matching finding region candidates comprises at least one of a representative slice image in which the matching finding region candidate is located in the second medical image, or a representative location of the matching finding region candidate in the representative slice image.

3. The method of claim 1, wherein the characteristic of each of the one or more matching finding region candidates comprises at least one of a correlation between a shape of the unmatched first finding region and a shape of the matching finding region candidate, or a correlation between clinical diagnosis information of the unmatched first finding region and clinical diagnosis information of the matching finding region candidate.

4. The method of claim 3, wherein the clinical diagnosis information of the unmatched first finding region and the clinical diagnosis information of the matching finding region candidate comprise a Reporting & Data System (RADS) category.

5. The method of claim 3, wherein the clinical diagnosis information of the unmatched first finding region and the clinical diagnosis information of the matching finding region candidate comprise distribution information about a distribution of signal intensity values within a finding region.

6. The method of claim 1, further comprising, when the user input for the unmatched first finding region is received, providing a user interface through which the user can select a matching command for the unmatched first finding region,
wherein the providing comprises, when a user input for the matching command is received via the user interface through which the matching command for the unmatched first finding region can be selected, providing the one or more matching finding region candidates to the user in response to the user input for the matching command.

7. The method of claim 1, further comprising, when an automatic matching process for the first finding regions and the second finding regions fails, visualizing the first finding region, unmatched with the second finding regions, among the first finding regions and providing it to the user.

8. The method of claim 1, further comprising:
providing the user with a first finding region, matched with any one of the second finding regions, among the first finding regions; and
providing a user interface through which the user can select an unmatching command for the matched first finding region.

9. The method of claim 8, wherein the receiving the user input comprises providing the user with the unmatched first finding region generated by the unmatching command.

10. An apparatus for assisting finding region matching in medical images, the apparatus comprising:
memory configured to store one or more instructions; and
a processor configured to execute the one or more instructions,
wherein the processor, by executing the one or more instructions, is configured to:
receive a second medical image generated at a different time and containing anatomical information about a body part of a patient that is the same as that of a first medical image;
receive a user input for a first finding region, unmatched with second finding regions identified in the second medical image, among first finding regions identified in the first medical image;
for the unmatched first finding region, provide a user with one or more matching finding region candidates among the second finding regions for matching with the unmatched first finding region; and
provide the one or more matching finding region candidates, sorted based on at least one of locations, sizes, or characteristics of the one or more matching finding region candidates, to the user.

11. The apparatus of claim 10, wherein the location of each of the one or more matching finding region candidates comprises at least one of a representative slice image in which the matching finding region candidate is located in the second medical image, or a representative location of the matching finding region candidate in the representative slice image.

12. The apparatus of claim 10, wherein the characteristic of each of the one or more matching finding region candidates comprises at least one of a correlation between a shape of the unmatched first finding region and a shape of the matching finding region candidate, or a correlation between clinical diagnosis information of the unmatched first finding region and clinical diagnosis information of the matching finding region candidate.

13. The apparatus of claim 12, wherein the clinical diagnosis information of the unmatched first finding region and the clinical diagnosis information of the matching finding region candidate comprise a Reporting & Data System (RADS) category.

14. The apparatus of claim 12, wherein the clinical diagnosis information of the unmatched first finding region and the clinical diagnosis information of the matching finding region candidate comprise distribution information about a distribution of signal intensity values within a finding region.

15. The apparatus of claim 10, wherein the processor, by executing the one or more instructions:
when the user input for the unmatched first finding region is received, provides a user interface through which the user can select a matching command for the unmatched first finding region; and
in providing the one or more matching finding region candidates to the user,
when a user input for the matching command is received via the user interface through which the matching command for the unmatched first finding region can be selected, provides the one or more matching finding region candidates to the user in response to the user input for the matching command.

16. The apparatus of claim 10, wherein the processor, by executing the one or more instructions, when an automatic matching process for the first finding regions and the second finding regions fails, visualizes the first finding region, unmatched with the second finding regions, among the first finding regions and provides it to the user.

17. The apparatus of claim 10, wherein the processor, by executing the one or more instructions:
provides the user with a first finding region, matched with any one of the second finding regions, among the first finding regions; and
provides a user interface through which the user can select an unmatching command for the matched first finding region.

18. The apparatus of claim 17, wherein the processor, by executing the one or more instructions, provides the user with the unmatched first finding region generated by the unmatching command.
